# FASCICULE DE BREVET EUROPEEN

(11) **EP 3 728 409 B1**
(45) Date de publication et mention de la délivrance du brevet: **06.10.2021**
(21) Numéro de dépôt: 18811274.2
(22) Date de dépôt: 04.12.2018
(51) Int. Cl.: C08H 8/00

(54) **PROCEDE DE TRAITEMENT DE BIOMASSE LIGNO-CELLULOSIQUE**
VERFAHREN ZUR BEHANDLUNG VON BIOMASSE AUS LIGNOCELLULOSE
METHOD FOR TREATING LIGNOCELLULOSIC BIOMASS

(30) Priorité: 20.12.2017 FR 1762610
(43) Date de publication de la demande: 28.10.2020
(73) Titulaire: IFP Energies nouvelles, 92852 Rueil-Malmaison (FR); Agro Industries Recherche Et Developpement, 51110 Pomacle (FR); Institut national de recherche pour l'agriculture, l'alimentation et l'environnement, 75338 Paris Cedex 07 (FR)
(72) Inventeur: AYMARD, Caroline, 69007 LYON (FR); PEROTTA, Larissa, 69007 LYON (FR)
(74) Mandataire: IFP Energies nouvelles
(86) Numéro de dépôt international: PCT/EP2018/083541
(87) Numéro de publication internationale: WO 2019/120995

(56) Documents cités:
- WO-A1-2015/173226
- WO-A1-2016/101073
- WO-A1-2017/088061
- WO-A1-2017/136915
- WO-A1-2018/015227
- CH-A5- 609 092

## Description

### Domaine de l'invention

L'invention concerne un procédé de traitement de biomasse lignocellulosique pour produire des jus sucrés dits de seconde génération (2G). Ces jus sucrés peuvent être utilisés pour produire d'autres produits par voie biochimique (ex : des alcools comme l'éthanol, le butanol ou d'autres molécules, par exemple des solvants tels que l'acétone etc...). Ce procédé comporte généralement trois étapes qui sont la préparation de liqueur, l'imprégnation de la biomasse et le prétraitement de la biomasse imprégnée, par exemple par cuisson, éventuellement suivie par une explosion à la vapeur. L'invention s'intéresse plus particulièrement aux deux premières étapes du procédé.

### Art antérieur

La biomasse lignocellulosique représente une des ressources renouvelables les plus abondantes sur terre. Les substrats considérés sont très variés, ils concernent à la fois des substrats ligneux comme différents bois (feuillus et résineux), des coproduits issus de l'agriculture (pailles de blé, pailles de riz, rafles de maïs, etc...) ou d'autres industries agroalimentaires, papetières, etc...

Le procédé de transformation biochimique de la lignocellulosique en jus sucrés 2G comprend notamment une étape de prétraitement et une étape d'hydrolyse enzymatique par un cocktail enzymatique. Ces procédés comportent aussi le plus souvent une étape d'imprégnation avant le prétraitement. Les jus sucrés issus de l'hydrolyse sont ensuite traités, par exemple par fermentation, et le procédé comprend également des étapes de séparation et/ou une étape de purification du produit final.

La biomasse lignocellulosique est composée de trois principaux polymères : la cellulose (35 à 50%), qui est un polysaccharide essentiellement constitué d'hexoses ; l'hémicellulose (20 à 30%), qui est un polysaccharide essentiellement constitué de pentoses ; et la lignine (15 à 25%), qui est un polymère de structure complexe et de haut poids moléculaire, composé d'alcools aromatiques reliés par des liaisons éther. Ces différentes molécules sont responsables des propriétés intrinsèques de la paroi végétale et s'organisent en un enchevêtrement complexe.

Parmi les trois polymères de base qui intègrent la biomasse lignocellulosique, la cellulose et l'hémicellulose sont ceux qui permettent la production de jus sucrés 2G.

Le plus souvent, l'hémicellulose est majoritairement décomposée en sucre durant le prétraitement et la cellulose est convertie en glucose par l'hydrolyse enzymatique. Toutefois, l'accès à la cellulose brute reste difficilement accessibles aux enzymes, d'où la nécessité d'un prétraitement. Ce prétraitement permet de modifier les propriétés physico-chimiques de la lignocellulosique afin d'améliorer l'accessibilité de la cellulose aux enzymes et sa réactivité à l'hydrolyse enzymatique.

De nombreuses technologies intéressant l'invention pour réaliser ce prétraitement existent, qui seront ci-après regroupés sous le terme générique de « cuisson » : cuissons acides, cuissons alcalines, explosion à la vapeur, procédés dits « organosolv pulping » selon le terme anglais connu (ou traitement avec organo-solvent en français). Ce dernier procédé concerne un prétraitement en présence d'un ou plusieurs solvants organiques et généralement d'eau. Le solvant peut être un alcool (éthanol), un acide type acide acétique, acide formique, ou encore l'acétone. Les procédés « organosolv pulping » conduisent à une solubilisation au moins partielle de la lignine, une solubilisation partielle des hémicelluloses. On a alors deux flux en sortie : le substrat prétraité avec cellulose, hémicellulose et lignine résiduels et la phase solvant qui contient la ligne solubilisée et une partie des hémicellulose. Il y a généralement une étape de régénération du solvant qui permet d'extraire un flux de lignine. Certains traitements « organosolv pulping » (notamment avec de l'éthanol) sont couplés avec l'ajout d'un acide fort (du type H2SO4). On peut envisager également d'avoir une mise en contact de la biomasse avec le solvant via un réacteur d'imprégnation avant la phase de cuisson ou d'avoir une mise en contact de la biomasse avec le catalyseur acide avant d'effectuer une cuisson « organosolv pulping ».

Différentes configurations sont reportées par exemple dans le document «Production of bioethanol from lignocellulosic materials via the biochemical pathway: A review », M. Balat, Energy Conversion and Management 52 (2011) 858-875, ou encore dans le document « Bioethanol production from agricultural wastes : an overview », N. Sarkar, S. Kumar Ghosh, S. Bannerjee, K. Aikat, Renwable Energy 37 (2012) 19-27.

Un des prétraitements le plus efficace est l'explosion à la vapeur en condition acide, qui permet une hydrolyse presque totale de l'hémicellulose et une amélioration importante de l'accessibilité et la réactivité de la cellulose aux enzymes. Ce prétraitement peut être précédé d'autre(s) traitement(s).

Les brevets US-8057639 et US-8512512 proposent un procédé comprenant une première étape d'hydrolyse de l'hémicellulose en sucres C5 dans des conditions douces les préservant ainsi de leur dégradation. Cette étape est réalisée dans un premier réacteur sous une pression de 1.5 bar ou plus par injection de vapeur, à une température de 110°C ou plus, et éventuellement en présence d'acide faible. Après cette étape, un lavage est réalisé pour extraire et récupérer les jus de sucres C5 avant d'envoyer la biomasse restante, enrichie en cellulose et lignine, dans une seconde étape (second réacteur) où a lieu l'explosion à la vapeur. Ce second réacteur opère à une pression plus élevée que le premier réacteur avec une injection de vapeur à pression élevée qui provoque une détente brutale de la biomasse (explosion à la vapeur).

La demande de brevet WO-2013/141776 décrit, dans le domaine papetier, un procédé d'imprégnation dans un réacteur (imprégnateur) vertical contenant de la liqueur basique d'imprégnation, définissant ainsi une première zone dans laquelle l'imprégnation est effectuée. La matière lignocellulosique est reçue en bas de l'imprégnateur, elle est transférée vers le haut de l'imprégnateur au moyen de deux vis de transfert. Lors de son transfert dans la seconde zone de l'imprégnateur située au-dessus du niveau du liquide, la biomasse s'égoutte et le liquide retombe dans la première zone. Dans cette configuration, le niveau de liquide est contrôlé par l'apport de liqueur basique.

L'invention a pour but d'améliorer le procédé d'imprégnation et l'outillage adapté décrits dans la demande WO-2013/141776 précitée.

L'invention vise notamment à améliorer la qualité de l'imprégnation de la biomasse avec une liqueur catalytique.

Accessoirement, l'invention cherche à rendre le procédé et l'outillage d'imprégnation plus simples à mettre en œuvre et/ou moins consommateurs en fluide.

### Résumé de l'invention

L'invention a tout d'abord pour objet un procédé de traitement d'une biomasse lignocellulosique, ledit procédé comprenant les étapes suivantes :
a. Préparation d'une liqueur d'imprégnation contenant un catalyseur chimique destiné à l'imprégnation de la biomasse, choisi parmi un catalyseur acide, un catalyseur basique et un catalyseur oxydant, de préférence un catalyseur acide
b. Introduction de la biomasse broyée par une entrée d'un réacteur d'imprégnation, ladite entrée étant située dans une première zone d'imprégnation dudit réacteur d'imprégnation qui comprend deux zones superposées , ladite première zone d'imprégnation et une deuxième zone dite d'égouttage au-dessus de la zone d'imprégnation
c. Introduction de la liqueur par une première entrée de liqueur située dans ladite première zone d'imprégnation du réacteur,

L'invention se caractérise en que le procédé comprend également l'étape suivante :
d. Introduction de ladite liqueur dans ledit réacteur par une deuxième entrée de liqueur dans une autre zone du réacteur située en dessous de l'entrée de la biomasse située dans la première zone d'imprégnation.

Dans le cadre de la présente invention, on comprend par les références spatiales de type « en dessus » ou « en dessous » en fonction de l'orientation préférentiellement verticale ou oblique par rapport à la verticale du réacteur, qui , sur sa hauteur, comprend donc une succession de zones que traverse la biomasse qui va alimenter le réacteur, dont une zone d'introduction puis une zone d'égouttage. La biomasse connait de préférence un mouvement ascendant dans le réacteur, la zone d'introduction se trouvant alors de faite « sous » la zone d'égouttage. (L'invention pourrait s'adapter à d'autres configurations du réacteur en adaptant alors la terminologie).

L'invention propose donc d'injecter dans le réacteur d'imprégnation la liqueur catalytique non seulement dans la zone d'imprégnation du réacteur, ce qui est conventionnel, mais aussi sous la zone d'imprégnation, en dessous du point d'injection de la biomasse dans le réacteur. Cette zone est généralement considérée comme une zone « morte » ou « inactive » du réacteur, puisque la biomasse introduite dans la première zone d'imprégnation va être amenée à la zone d'égouttage, au-dessus de la zone d'imprégnation, ce mouvement ascendant étant provoqué par des moyens de transport dans le réacteur du type vis de transport par exemple. Cette zone, généralement de faible hauteur par rapport aux deux autres et qui se trouve en dessous de la première zone d'imprégnation, correspond au fond du réacteur, généralement disposé selon la verticale ou l'oblique, fond par lequel est(sont) introduite(s) dans le réacteur la ou les vis de transport dont le mouvement est actionné par un moteur Injecter de la liqueur dans cette zone allait à l'encontre des habitudes puisque peu/pas de biomasse séjourne dans cette zone, et s'il était envisagé d'injecter un fluide dans cette zone, ce n'était éventuellement que de l'eau destinée, dans le cas de l'utilisation de vis de transport dans le réacteur, à lubrifier les vis, dans un but essentiellement mécanique donc.

Or, il s'est avéré, de manière surprenante, qu'injecter de la liqueur catalytique dans cette zone, sous le point d'injection de la biomasse dans le réacteur, était particulièrement bénéfique au procédé d'imprégnation. Il a en effet été prouvé, de façon indirecte, par la mesure comparée des rendements à l'issue du prétraitement par explosion à la vapeur suivant le traitement par imprégnation, que l'imprégnation de la biomasse était mieux réalisée, à consommation en catalyseur égale (c'est-à-dire sans surconsommer de liqueur catalytique, mais en l'injectant en deux zones différentes du réacteur au lieu d'une).

Les raisons de cette meilleure imprégnation pourraient être multiples : la liqueur injectée sous le point d'injection de la biomasse pourrait permettre de mieux traiter la biomasse juste dans sa zone d'injection. En effet, pour injecter la biomasse, on peut usuellement recourir à une vis d'alimentation qui vient comprimer et amener la biomasse dans le réacteur, un bouchon de biomasse se formant en partie avale de la vis qui vient empêcher toute remontée de liquide vers l'amont de la vis. Et ce bouchon, quand il pénètre dans le réacteur, va s'ouvrir, notamment de façon mécanique par l'utilisation d'un piston qui vient, dans le réacteur, à la rencontre du bouchon en question. Injecter de la liqueur juste sous la zone où ce bouchon de biomasse vient s'éclater parait extrêmement propice à une imprégnation par le catalyseur plus « tôt » de la biomasse dans son cheminement dans le réacteur, donc à une imprégnation meilleure, plus complète, à temps de séjour identique dans le réacteur. En outre, on a pu constater que la liqueur ainsi injectée remplace parfaitement une éventuelle eau de lavage pour lubrifier les moyens de transport de la biomasse dans le réacteur, du type vis.

D'autres avantages découlent de l'invention : On ne consomme pas plus de catalyseur pour un meilleur résultat. On vient simplifier l'outillage, puisqu'au lieu de prévoir une amenée d'eau indépendante en bas de réacteur, on vient prélever de la liqueur depuis sa zone de préparation. Limiter l'ajout d'eau dans le réacteur est également avantageux pour limiter l'effet de dilution de la liqueur catalytique dans le réacteur, pour limiter le besoin éventuel, donc, de réajuster le taux de catalyseur de la liqueur par exemple.

Avantageusement, et comme précédemment évoqué, la biomasse est transportée de la première zone d'imprégnation à la deuxième zone d'égouttage par des moyens de transport du type une ou des vis de transport. La biomasse s'imprègne de liqueur dans la première zone et s'égoutte dans la deuxième zone. La vis de transport de la zone d'égouttage peut être entourée d'une grille par les orifices desquels s'écoule le liquide en excès.

Avantageusement encore, l'introduction de la biomasse dans la première zone d'imprégnation du réacteur est effectuée par un moyen d'alimentation créant un bouchon de biomasse empêchant la remontée de liquide depuis ladite première zone dans ledit moyen d'alimentation, ledit moyen d'alimentation étant notamment une vis d'alimentation.

Selon l'invention, on va donc injecter la liqueur provenant de la même zone de préparation en deux points, et on va pouvoir, à consommation de liqueur donnée, doser la proportion de liqueur injectée dans la zone d'imprégnation par rapport à celle injectée sous le point d'injection de la biomasse : de préférence, 80 à 98%, notamment 85 à 90%, (en volume) de la liqueur introduite dans le réacteur d'imprégnation est introduite par la première entrée de liqueur, et le complément à 100% de la liqueur introduite dans le réacteur est introduite par la deuxième entrée de liqueur. On injecte donc la plus grande partie de la liqueur dans sa zone conventionnelle, « en prélevant » jusqu'à 20% de cette quantité pour l'injecter en fond de réacteur.

L'introduction de liqueur par la première entrée de liqueur et/ou par la deuxième entrée de liqueur peut se faire de façon continue ou discontinue.

De préférence, l'autre zone du réacteur dans laquelle est située la deuxième entrée de liqueur est une zone inactive située dans le fond du réacteur disposé sensiblement à la verticale ou à l'oblique par rapport à la verticale.

Selon un mode de réalisation préféré, la liqueur utilisée est une liqueur à catalyse acide, et on règle le pH de la liqueur entre 0,1 et 4, notamment entre 0,3 et 2.

Comme acide, on peut par exemple utiliser au moins un acide choisi parmi l'acide sulfurique, l'acide chlorhydrique, l'acide nitrique, l'acide oxalique. Leur teneur, en phase aqueuse, est de préférence comprise entre 0,2 et 8% poids.

L'invention a également pour objet un procédé de traitement en continu d'une biomasse lignocellulosique, comprenant les étapes précédemment décrites, et qui se poursuit, en continu ou non, en comprenant les étapes suivantes :
e. Transfert de la biomasse imprégnée et égouttée depuis la sortie du réacteur d'imprégnation vers un réacteur de cuisson, éventuellement couplée à une explosion à la vapeur
f. Prétraitement de ladite biomasse dans ledit réacteur
g. Hydrolyse enzymatique de la biomasse prétraitée
h. Fermentation alcoolique du moût d'hydrolyse enzymatique obtenu à partir de la biomasse prétraitée.

L'invention a également pour objet une installation de traitement d'une biomasse lignocellulosique, comprenant :
- Une zone de préparation d'une liqueur d'imprégnation contenant un catalyseur chimique pour l'imprégnation de la biomasse, notamment un catalyseur acide, basique ou oxydant, et de préférence acide et munie d'une sortie de liqueur
- Un réacteur d'imprégnation comprenant une première zone d'imprégnation munie d'une entrée de biomasse et une deuxième zone superposée à la première, dite zone d'égouttage, et munie d'une sortie de biomasse
- Un moyen d'alimentation en biomasse broyée du réacteur d'imprégnation par l'entrée de biomasse du réacteur située dans la première zone d'imprégnation
- Un premier moyen d'alimentation en liqueur d'imprégnation du réacteur reliant une sortie en liqueur de la zone de préparation de la liqueur à une première entrée de liqueur dans la première zone d'imprégnation du réacteur,
telle que l'installation comprend également :
- Un deuxième moyen d'alimentation en liqueur d'imprégnation du réacteur reliant une sortie en liqueur de la zone de préparation de la liqueur à une deuxième entrée de liqueur dans une zone du réacteur située en dessous de l'entrée de la biomasse de la première zone d'imprégnation.

Cette installation permet de mettre en œuvre le procédé décrit précédemment, en prévoyant les moyens pour introduire dans un deuxième point du réacteur d'imprégnation la liqueur catalytique.

A noter que l'invention inclut aussi l'injection de liqueur catalytique dans le réacteur d'imprégnation en plus de deux points d'injection, par exemple par trois ou quatre entrées distinctes dans le réacteur, dont au moins une, par exemple deux, dans la zone d'imprégnation et au moins une, par exemple une ou deux, sous le point d'injection de la biomasse

L'installation selon l'invention utilise de préférence un réacteur d'imprégnation d'orientation verticale ou oblique par rapport à la verticale, la biomasse effectuant un mouvement ascendant depuis la première zone d'imprégnation vers la deuxième zone d'égouttage par une ou des vis de transport disposées dans le réacteur dans lesdites zones.

La zone de préparation de la liqueur d'imprégnation peut être une cuve, ou un mélangeur statique ou un mélangeur dynamique ou tout autre moyen permettant de mélanger un solvant (par exemple de l'eau) et le catalyseur (par exemple un acide du type H₂SO₄ dans le cas d'une liqueur acide).

Selon un premier mode de réalisation, le premier et le deuxième moyens d'alimentation en liqueur comprennent une portion de conduite commune reliée à une même sortie de liqueur de la zone de préparation de la liqueur : on vient faire une dérivation sur la conduite d'amenée de la liqueur vers la zone d'imprégnation du réacteur.

Selon un autre mode de réalisation, le premier et le deuxième moyens d'alimentation en liqueur sont reliés chacun à une sortie distincte de la zone de préparation de la liqueur : on utilise alors une conduite complètement distincte pour acheminer la liqueur depuis une autre sortie vers la deuxième entrée de liqueur du réacteur.

Comme déjà évoqué, le moyen d'alimentation en biomasse est conçu de préférence pour créer un bouchon de biomasse empêchant la remontée de liquide depuis ladite première zone dans ledit moyen d'alimentation, ledit moyen d'alimentation étant notamment une vis d'alimentation.

L'installation selon l'invention peut aussi comprendre également un réacteur de prétraitement de la biomasse obtenue en sortie du réacteur d'imprégnation, un réacteur d'hydrolyse enzymatique et un réacteur de fermentation alcoolique, l'ensemble des réacteurs, ou au moins deux d'entre eux, étant montés en série.

L'invention a également pour objet l'utilisation du procédé ou de l'installation précédemment décrits pour le traitement de biomasses du type bois, paille, résidus agricoles, et toutes cultures énergétiques dédiées, notamment des plantes annuelles ou pluriannuelles telles que le miscanthus vue de produire des sucres, des biocarburants ou des molécules biosourcées. De façon plus générale, la biomasse lignocellulosique, ou matériaux lignocellulosiques employés dans le procédé selon l'invention est obtenue par exemple à partir de bois (feuillus et résineux), brut ou traité, de sous-produits de l'agriculture tels que la paille, de fibres de plantes, de cultures forestières, de résidus de plantes alcooligènes, sucrières et céréalières, de résidus de l'industrie papetière, de biomasse marine (par exemple macroalgues cellulosiques) ou de produits de transformations de matériaux cellulosiques ou lignocellulosiques. Les matériaux lignocellulosiques peuvent également être des biopolymères et sont préférentiellement riches en cellulose.

Préférentiellement, la biomasse lignocellulosique utilisée est du bois, de la paille de blé, de la pulpe de bois, du miscanthus, de la paille de riz ou des tiges de maïs.

### Description détaillée

L'invention sera ci-après détaillée à l'aide d'exemples non limitatifs illustrés par les figures suivantes :
- Figure 1 : une représentation schématique d'un outillage d'imprégnation de biomasse par une liqueur selon l'art antérieur ;
- Figure 2 : une représentation synoptique, de type schéma-bloc, de l'outillage et du procédé d'imprégnation mis en œuvre dans l'invention ;
- Figure 3 : une représentation synoptique, de type schéma-bloc, de l'outillage et du procédé complet depuis l'imprégnation de la biomasse comme représenté à la figure 2 jusqu'à la conversion de la biomasse en éthanol en continu.

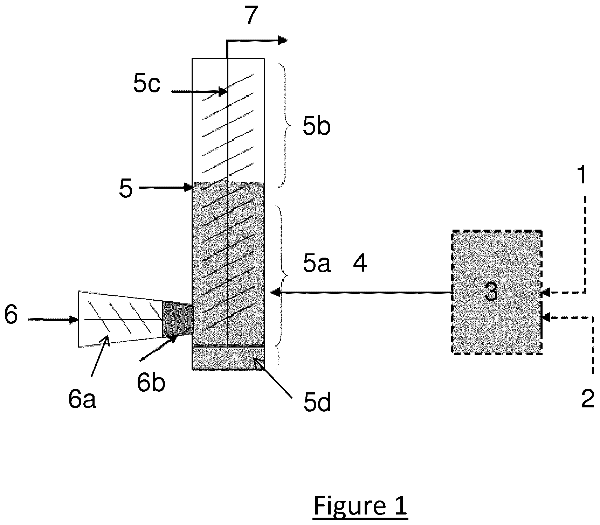

Description des figures 1 à 3 à partir des références suivantes, les mêmes références correspondant aux mêmes composants / fluides / produits sur l'ensemble des figures :
- 1 : Entrée d'eau dans la cuve de préparation de la liqueur
- 2 : Entrée d'acide dans la cuve de préparation de la liqueur
- 3 : Outil (cuve) de préparation de la liqueur
- 4 : Liqueur acide
- 4a : Liqueur acide vers outil (réacteur) d'imprégnation (injection par la zone d'imprégnation)
- 4b : Liqueur acide vers outil (réacteur) d'imprégnation (injection en fond pour lubrification des vis de convoyage)
- 5 : Outil (réacteur) d'imprégnation
- 5a : Zone d'imprégnation du réacteur d'imprégnation
- 5b : Zone d'égouttage du réacteur d'imprégnation
- 5c : Vis de transport de la biomasse dans le réacteur d'imprégnation
- 5d : zone du réacteur d'imprégnation située sous la zone 5a d'imprégnation (sous le point d'entrée de la biomasse 6b dans le réacteur 5 par la vis d'alimentation 6a)
- 6 : Biomasse broyée
- 6a : Vis conique d'alimentation en biomasse du réacteur d'imprégnation
- 6b : bouchon d'étanchéité de biomasse dans la vis d'alimentation 6a
- 7 : Biomasse imprégnée et égouttée
- 8 : Vis (ou « plug screw feeder » en anglais) de l'outil de prétraitement
- 9 : Outil (réacteur d'explosion) de prétraitement
- 10 : Pressât de la vis 8 de l'outil de prétraitement
- 11 : Injection de vapeur pour le prétraitement
- 12 : Biomasse prétraitée et vapeur
- 13 : Outil (cyclone) de séparation vapeur et biomasse prétraitée
- 14 : Vapeur vers condensation
- 15 : Biomasse prétraitée
- 16 : Hydrolyse enzymatique
- 17 : Moût d'hydrolyse enzymatique contenant des sucres
- 18 : Fermentation alcoolique (éthanolique)
- 19 : Vin de fermentation contenant de l'éthanol (alcool)
- 20 : Distillation
- 21 : Alcool concentré
- 22 : Vinasses brutes

**La** **figure 1** est une représentation schématique d'un outillage d'imprégnation de biomasse par une liqueur selon l'art antérieur, elle n'est donc pas nécessairement à l'échelle et ne représente pas tous les composants de l'outillage, mais seulement ceux intéressant plus particulièrement la description de l'invention.

Le procédé selon l'invention est un procédé de traitement d'une biomasse lignocellulosique avant hydrolyse enzymatique. Il s'insère dans les procédés visant à produire des sucres de deuxième génération à partir desquels de nombreuses voies biochimiques permettent d'obtenir des molécules oxygénées (par exemple des alcools comme éthanol, butanol...).

Ainsi les exemples suivants concernent un procédé intégré d'imprégnation acide suivi, en continu ou non, d'un prétraitement par explosion à la vapeur, avec éventuellement un recyclage de la liqueur acide d'imprégnation.

Ce procédé est compatible avec les procédés de production de sucres 2G (c'est-à-dire obtenus à partir de biomasse lignocellulosique) ou plus largement de molécules bio-sourcées (c'est-à-dire à partir de substrats naturels ou dérivés de substrats naturels).

### La biomasse et la zone de transfert

Selon les biomasses (pailles, bois, etc...), une étape de broyage est nécessaire pour avoir une granulométrie compatible avec les moyens technologiques et les conditions opératoires des étapes. Pour cela, un simple déchiquetage peut être suffisant mais un broyage avec ou sans affinage peut être requis.

De façon générale, la biomasse broyée 6 présente une taille de particule (la plus grande taille) d'au plus 300 mm. Le plus souvent, le broyage des pailles se fait avec des grilles de 5 à 100 mm et le bois est déchiqueté en plaquettes parallélépipédiques avec une longueur comprise entre 20 et 160 mm, une largeur comprise entre 10 et 100 mm et une épaisseur comprise entre 2 et 20 mm.

La biomasse broyée 6 est amenée à la première zone d'imprégnation 5a du réacteur d'imprégnation via une vis d'alimentation conique 6a dans laquelle un bouchon 6b hermétique de biomasse empêche la remontée de liquide de ladite zone 5a dans la vis 6a, juste avant l'entrée dans la première zone d'imprégnation. Cette vis conique de compression peut comprendre un carénage sous forme d'une grille perforée. Elle présente une partie de forme conique, ladite partie conique étant reliée en partie inférieure de la première zone d'imprégnation 5a du réacteur. Cette vis 6a assure ainsi une double fonction : d'une part, l'introduction de la biomasse de façon continue dans le réacteur d'imprégnation, et d'autre part la formation d'un bouchon pour faire l'étanchéité et empêcher les fuites de liqueur depuis le réacteur d'imprégnation vers la vis et l'amont de la vis.

### L'étape d'imprégnation

L'imprégnation est réalisée sous pression atmosphérique et à une température de 10-95°C. Le temps de séjour de la biomasse dans le réacteur d'imprégnation 5 est habituellement de 20secondes à 60 mn, de préférence d'au moins 30 secondes, de préférence d'au moins 1 mn, de préférence d'au plus 45mn, et le plus souvent entre 1 et 35mn. De préférence, elle est effectuée dans un seul réacteur.

Le réacteur d'imprégnation 5 (ou imprégnateur) est de forme tubulaire et d'orientation verticale ou inclinée avec un angle inférieur à 60° par rapport à la verticale. Il est dans les exemples vertical. Ce réacteur comporte deux zones d'imprégnation superposées 5a,5b, situées selon un même axe. La zone inférieure 5a est appelée première zone d'imprégnation et reçoit par une ouverture la biomasse pressée issue de la vis d'alimentation 6a, ouverture au-dessus de laquelle est prévue une arrivée 4 de liqueur acide toujours dans cette première zone 5a. La zone 5b située au-dessus (zone supérieure) est appelée deuxième zone d'imprégnation, ou zone d'égouttage : elle reçoit la biomasse provenant de la première zone d'imprégnation 5a.

Le réacteur 5 (imprégnateur) est muni de une ou plusieurs vis de transport 5c qui transfère(nt) la biomasse par le bas de la première zone d'imprégnation vers l'ouverture de sortie par le haut de la deuxième zone d'imprégnation 5b.

La première zone d'imprégnation 5a (donc la zone où a lieu l'imprégnation) correspond à l'espace rempli par la liqueur d'imprégnation. La seconde zone d'imprégnation 5b ne contient pas de phase liquide continue. Il est particulièrement avantageux de maintenir une répartition constante entre la première zone d'imprégnation et la seconde zone d'imprégnation. Pour ce faire, le réacteur est équipé d'un système de détection (capteur de niveau), avec de préférence un système de régulation du niveau de liqueur (non représentés), permettant de garantir un remplissage au niveau souhaité.

La liqueur catalytique d'imprégnation est une solution aqueuse ayant un pH de 0.1 à 6, de référence de 0.2 à 4.0, et une température de 10-95°C. L'acide est ici de l'acide sulfurique. Ce type de liqueur est bien connu de l'homme du métier et tout autre acide habituellement utilisé pour l'imprégnation convient. La quantité d'acide et la température de la liqueur sont généralement fixées. Les moyens pour obtenir et maintenir la température sont connus de l'homme du métier. La préparation de la liqueur se fait ici dans une cuve 3 avec une entrée d'eau 1 et une entrée d'acide sulfurique concentrée 2.

L'effet de compression de la biomasse lors de la formation du bouchon (au niveau de la vis de transfert) et de décompression en entrée de la première zone d'imprégnation remplie de liqueur permet de mieux gorger la biomasse (effet d'éponge). La biomasse est transférée à travers la première zone 5a où elle est imprégnée vers la seconde zone d'imprégnation 5b située au-dessus du niveau de la liqueur.

Dans la deuxième zone d'imprégnation 5b, une partie de la liqueur imprégnée se sépare de la biomasse imprégnée par égouttage durant l'ascension dans la seconde zone d'imprégnation, la liqueur égouttée retombant dans la première zone d'imprégnation 5a.

De préférence, la deuxième zone d'imprégnation 5b est munie de grille(s) retenant la biomasse humide dans la seconde zone d'imprégnation, grille qui laisse donc s'écouler le liquide de la deuxième zone d'imprégnation 5b dans la première zone d'imprégnation 5a.

En sortie de la seconde zone d'imprégnation et du réacteur d'imprégnation, la biomasse imprégnée et égouttée est récupérée et contient peu ou pas d'eau libre. Sa teneur en matière sèche varie généralement entre 15 et 40% en poids.

La liqueur séparée, appelée souvent liqueur usée, se retrouve au niveau du liquide de la première zone d'imprégnation.

### La préparation de la liqueur d'imprégnation

Du fait de l'imprégnation, il y a une perte de liqueur et d'acidité. Il est donc nécessaire d'ajouter régulièrement de la liqueur fraîche acide.

Ces ajouts permettent de réguler précisément le niveau de liqueur dans le réacteur d'imprégnation.

La préparation de liqueur est aussi une étape qui permet de réguler ses paramètres opératoires comme, par exemple, la température, le pH ou toute autre caractéristique. La concentration acide adéquate est réglée grâce à des appoints en acide et/ou en eau. Elle permet également de produire une liqueur homogène. Cette étape est réalisée dans une zone de préparation de liqueur.

On peut employer des appareils variés comme, par exemple ici, une cuve de mélange munie d'un système d'agitation ou un mélangeur non représentés (de préférence un mélangeur statique).

De préférence, l'appareil est équipé de capteurs de mesure de pH et de débit pour l'eau, l'acide, et la liqueur préparée.... L'ensemble de ces capteurs permet de mettre en place une régulation qui équilibre les débits et les acidités de manière à avoir une marche continue stable aux conditions souhaitées.

La cuve 3 de préparation de la liqueur et/ou le réacteur d'imprégnation 5 est (sont) équipé(s) pour réaliser le chauffage, de moyens de chauffage, comme une double enveloppe, des serpentins (et éventuellement des échangeurs disposés sur la boucle de recirculation optionnelle de la liqueur, à côté ou directement sur lesdits appareils (cuve, mélangeur...).

La cuve 3 de la liqueur est reliée au réacteur d'imprégnation par une ou plusieurs conduites transportant la liqueur.

La liqueur peut ainsi être préparée avec la concentration et le débit adéquats qui permettent d'obtenir le pH (ou toute autre caractéristique) déterminé qui peut être la valeur consigne pour la régulation.

L'invention est née de la constatation suivante : toujours dans la figure 1, dans le réacteur d'imprégnation 5, on note l'existence d'une 3^{e} zone 5d située en dessous du niveau d'introduction du bouchon de biomasse 6b, en dessous de la première zone d'imprégnation 5a: il s'agit d'une zone dite « morte », car il n'y a pas de passage de biomasse souhaité à cet endroit. Cette zone morte ou « inactive » est nécessaire pour laisser de l'espace pour l'expansion du bouchon et pour limiter l'accumulation de solides en fond de réacteur. Cette zone 5d est constituée essentiellement de liquide, puisqu'elle se situe en dehors du passage souhaité pour le solide. Néanmoins, en raison de déviations à l'idéalité d'un transport piston par les vis ascendantes 5c, et de la présence de particules dans le bouchon de biomasse, on observe la présence possible d'une faible fraction de solides dans la zone morte. Or il est souhaitable d'éviter l'accumulation de composants solides dans cette zone 5d.

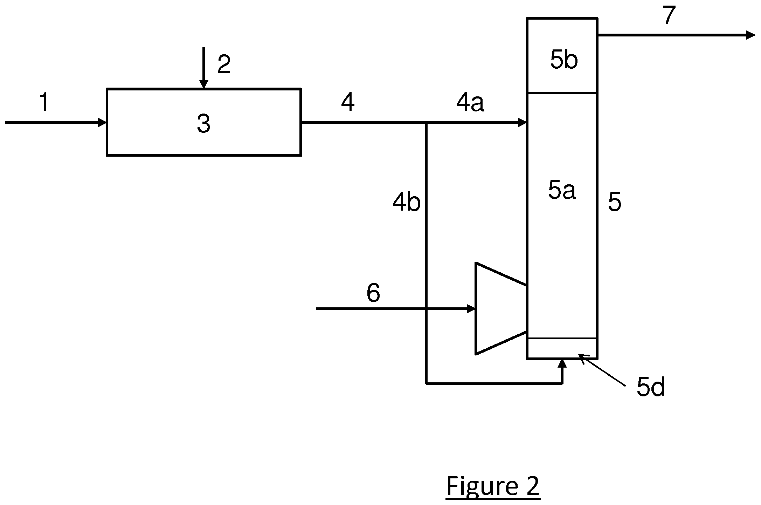

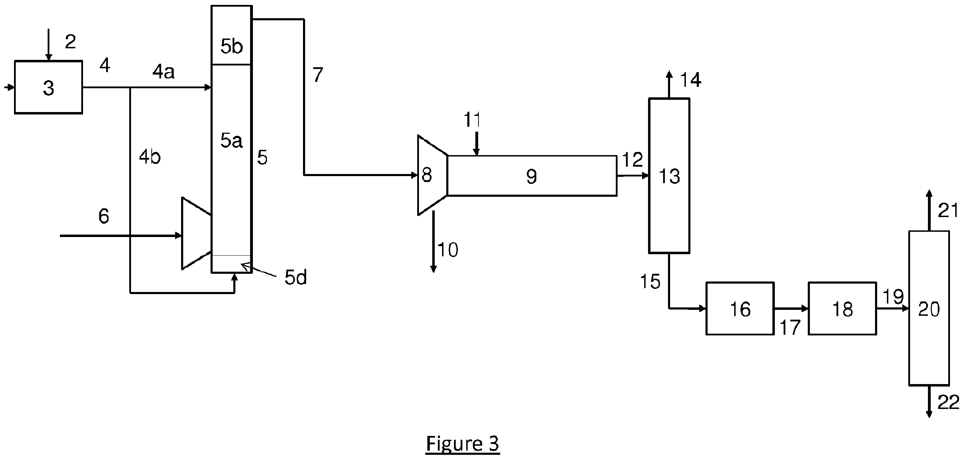

Il a alors été choisi d'injecter dans cette zone 5d un fluide, non pas une simple arrivée d'eau qui viendrait provoquer une dilution supplémentaire de la liqueur dans le réacteur, mais une arrivée de liqueur supplémentaire provenant de la cuve de préparation 3, représentée aux figures 2 et 3 ci-après décrites.

**Selon la** **figure 2****,** l'acide et l'eau sont introduits respectivement par les conduites 1 et 2 dans la cuve de préparation de la liqueur acide 3. La liqueur acide de la conduite 4 est ensuite injectée dans l'imprégnateur 5 pour être mélangée avec la biomasse broyée qui est introduite par la conduite 6 dans le procédé. La liqueur acide 4 est injectée dans la cuve d'imprégnation 5, et plus précisément dans la zone d'imprégnation 5a, par le biais des conduites 4a et 4b. La biomasse - mise en contact avec la liqueur acide dans la zone d'imprégnation 5a - passe ensuite dans la zone d'égouttage 5b afin d'être séparée de la fraction liquide. La biomasse imprégnée et égouttée est transférée par la conduite 7 vers l'étape suivante du procédé.

Dans cet exemple de réalisation de l'invention, il a été choisi d'ajouter une conduite d'amenée 4b supplémentaire de liqueur dans la zone 5d, sous le point d'alimentation en biomasse du réacteur 5, ici sous la forme d'une conduite en dérivation de la conduite principale 4-4a reliée à une entrée de la cuve 3. Naturellement, toute autre disposition de conduites pour acheminer également de la liqueur au fond du réacteur est également possible, notamment en prévoyant alternativement une conduite dédiée reliant une deuxième sortie de la cuve à l'entrée en zone 5d du réacteur 5, ou en mettant en œuvre tout autre système de distribution de liqueur depuis la cuve 3 jusqu'à deux entrées distinctes du réacteur 5. Des moyens pour ajuster la quantité de liqueur à injecter en zone d'imprégnation 5a par rapport à la quantité à injecter dans la zone 5d sont prévus et connus de l'état de l'art (vannes régulées...)

**A la** **figure 3****,** est représenté le procédé de la figure 2, se poursuivant au-delà de l'imprégnation par un prétraitement par explosion de la vapeur en continu, puis par plusieurs autres étapes de traitement successives jusqu'à la fermentation alcoolique.

Le procédé se déroule donc de la façon suivante : l'imprégnation se fait comme à la figure 2 dans le réacteur 5, et ne sera donc pas décrite à nouveau.

La biomasse une fois imprégnée et égouttée est transférée par la conduite 7 est introduite dans l'unité de prétraitement par explosion à la vapeur 9 au moyen d'une vis de transport 8 qui permet de comprimer la biomasse pour former un bouchon de biomasse Lors de cette compression, une séparation solide/liquide a lieu, et la liqueur acide usée 10 est évacuée à travers la zone perforée de la vis. Optionnellement, cette liqueur acide usée 10, appelée aussi pressât, peut être recyclée au moins en partie en la réintroduisant dans la cuve de préparation de la liqueur 3.La biomasse est ensuite traitée dans l'outil de traitement 9. La vapeur est introduite par la conduite 11 dans le réacteur de prétraitement 9. Dans ce réacteur est opérée une cuisson acide, couplée à une explosion à la vapeur. Une sortie par détente explosive a lieu et le mélange de substrat prétraité et de vapeur 12 est envoyé vers d'un outil de séparation type cyclone 13.

A noter que, dans cet exemple, la cuisson est suivie d'une explosion à la vapeur, mais que l'explosion à la vapeur reste optionnelle, et que d'autres traitements que la cuisson sont possibles pour atteindre l'objectif de ce prétraitement, à savoir les modifications d'au moins une propriété physico chimique de la biomasse (imprégnée), comme son degré de polymérisation ou sa cristallinité. Ces autres traitements de cuisson ont été mentionnés plus haut. Les conditions opératoires de la cuisson, ici acide, sont par exemple les suivantes : l'outil 9 comprend une zone de cuisson, dans laquelle la biomasse est mise en contact pendant 1-30mn avec de la vapeur avec une consommation spécifique de vapeur de 0.05-10 tonne/tonne de matière sèche de biomasse, ladite zone étant à une température de 150-250°C et une pression de 0.5-4 MPa, puis une zone de détente de la biomasse issue de la zone de cuisson , puis une zone de séparation de la vapeur de la biomasse. De façon préférée, les conditions sont réglées de façon à ce que le temps de cuisson soit limité à 1-30mn. Généralement la consommation spécifique de vapeur est de 0.05-10 tonne/tonne de matière sèche. La vapeur récupérée est avantageusement recyclée après compression à l'étape d'explosion à la vapeur, ou éventuellement est recyclée sur les utilités du site. Ici cette étape est réalisée dans un réacteur 9 qui est tubulaire et horizontal (qui peut aussi être légèrement incliné pour l'écoulement du liquide), et qui est muni d'une vis de transfert de biomasse. A l'extrémité avale du réacteur 9, la biomasse est entraînée très rapidement par la vapeur vers une zone de détente dans une ligne appelée « blowline » qui a un diamètre réduit par rapport à la zone de cuisson.

Dans l'outil 13, la vapeur 14 est séparée de la biomasse prétraitée 15. La biomasse prétraitée est ensuite transformée dans l'outil de transformation 16 en un moût 17 contenant des sucres en utilisant un cocktail enzymatique. Les sucres sont convertis en alcool (ex : éthanol, acétone, butanol) par fermentation dans l'étape 18 de fermentation. Le vin de fermentation 19 est envoyé vers une étape de séparation et purification 20. L'étape 20, réalisée par exemple par distillation, permet de séparer un flux 21 contenant l'alcool concentré des vinasses brutes (eau usée, lignine résiduelle) 22. Les conditions de l'hydrolyse enzymatique et de la fermentation, qui peuvent être consécutives ou simultanées, sont adaptées aux produits souhaités et sont connues de l'homme du métier.

A noter, comme pour la figure 2, que l'invention s'applique de la même manière et avec les mêmes avantages sur un procédé où certaines ou toutes les étapes suivant l'explosion à la vapeur se font non pas en continu mais en « batch » ou en « batch alimenté ».

### Exemples

Dans les exemples décrits ci-après, l'acronyme "MS" désigne le taux de matière sèche qui est mesurée selon la norme ASTM E1756 - 08(2015) « Standard Test Method for Détermination of Total Solids in Biomass".

### Exemple 1 (comparatif)

Cet exemple concerne un procédé d'imprégnation et de prétraitement de bois avec les caractéristiques suivantes :
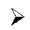 Charge : Bois peuplier, débit 6,79 tonne / heure, teneur en MS : 55,6%, composition moyenne (base MS) :

| | % (base MS) |
|---|---|
| Cellulose | 42,6% |
| Hémicellulose | 20,5% |
| Lignine et autres (cendres, extractibles, etc...) | 36,9% |

Le bois est utilisé sous forme de plaquettes de dimension caractéristique 50 mm. La température des plaquettes en entrée d'unité est la température ambiante.

Les plaquettes sont convoyées vers le réacteur d'imprégnation 5 par la vis conique 6a. Le réacteur d'imprégnation 5 est un tube vertical dans lequel la biomasse 6b est convoyée verticalement par deux vis 5c en série tournant en sens de rotation inverses. Le volume utile total du réacteur est de 1,95 m³. La vitesse des vis et le niveau liquide sont réglés de sorte à assurer un temps de séjour de 30 secondes dans la zone immergée et un temps d'égouttage de 60s.

Dans le réacteur d'imprégnation 5 sont introduites :
- les plaquettes de bois compressées, via la vis de transfert conique 6a
- 2,5 tonne / heure d'une liqueur acide préparée avec de l'eau et de l'acide sulfurique en concentration massique 1,76%pds, à une température de 90°C
- 0,3 tonne / heure d'eau introduite en fond de réacteur d'imprégnation 5 dans la zone 5d pour lubrifier les vis de transport vertical 5c, à une température de 20°C

Les plaquettes imprégnées sont sorties du réacteur d'imprégnation 5 et transférées vers le réacteur 9 d'explosion à la vapeur. Le prétraitement par explosion à la vapeur est effectué à 200°C dans une configuration continue décrite plus haut et employant un court temps de séjour. Le milieu est brusquement détendu à une pression de 1,3 atm.

Le substrat prétraité ainsi obtenu a une teneur en xylose libre de 6,9% de xylose (base MS), et une teneur en xylose potentiel (monomère, oligomères et polymères) de 14,5% exprimé sous forme de xylose (base MS). L'imprégnation a consommé 44 kg d'H₂SO₄ pur /h, soit 11,65 kg d'acide pur / tonne MS de substrat. Le taux de libération du xylose, exprimé comme le pourcentage de xylose sous forme libre sur le total de xylose potentiel, est de 47,6%.

### Exemple 2 (selon l'invention)

Cet exemple utilise un procédé d'imprégnation selon l'invention et de prétraitement de bois traitant la même charge que l'exemple 1.

Les plaquettes sont convoyées vers le réacteur d'imprégnation 5 par la vis conique 6a. Le réacteur d'imprégnation 5 est le même qu'à l'exemple 1.La vitesse des vis 5c et le niveau liquide sont réglés de sorte à assurer un temps de séjour de 30 secondes dans la zone immergée et un temps d'égouttage de 60s. Dans le réacteur d'imprégnation 5 sont introduites :
- les plaquettes de bois compressées, via la vis de transfert conique 6a
- 2,5 tonne / heure d'une liqueur acide préparée avec de l'eau et de l'acide sulfurique en concentration massique 1,57% en poids, à une température de 85°C, par une entrée située dans la zone d'imprégnation 5a
- 0,3 tonne / heure de la même liqueur acide est introduite en fond de réacteur d'imprégnation 5 ; dans la zone 5d sous le point d'injection de la biomasse dans le réacteur 6, à une température de 85°C

Les plaquettes imprégnées sont sorties du réacteur d'imprégnation 5 et sont transférées en continu vers un réacteur d'explosion à la vapeur 9 via la vis d'alimentation 8. Le prétraitement par explosion à la vapeur est effectué de la même manière que pour l'exemple 1 comparatif

Le substrat prétraité ainsi obtenu à une teneur en xylose libre de 11,1% de xylose (base MS), et une teneur en xylose potentiel (monomère, oligomères et polymères) de 15,1% exprimé sous forme xylose (base MS). L'imprégnation a consommé 44kg d'H₂SO₄ pur /h, soit 11,65 kg d'acide pur / tonne MS de substrat. Le taux de libération du xylose, exprimé comme le pourcentage de xylose sous forme libre sur le total de xylose potentiel, est de 73,5%.

Il n'existe pas de mesure précise et robuste de la qualité d'imprégnation de la biomasse par la liqueur en sortie de l'étape d'imprégnation, mais cette qualité peut être appréciée, indirectement, par le taux de xylose obtenu à la fin du prétraitement par explosion à la vapeur dans le réacteur 9 selon la figure 2. A noter que les avantages de l'invention sont préservés également si l'étape d'imprégnation et l'étape de pré traitement par explosion à la vapeur sont opérées de façon discontinue, par « batch ».

Et en comparant les résultats des exemples 1 et 2, on voit que l'invention, en injectant aussi de la liqueur en fond de réacteur a permis d'obtenir une bien meilleure libération du xylose dans le substrat pré traité, toutes choses égales par ailleurs, démontrant ainsi une meilleure imprégnation de la biomasse, à consommation d'acide identique : on passe notamment de 6,9% de taux de xylose libre à 11,1% de taux de xylose libre, ce qui veut dire qu'avec l'invention on arrive à libérer près de deux fois plus de xylose à partir de la même biomasse sans consommer plus d'acide, avec une modification dans le procédé et les outillages tout à fait modeste et faciles à mettre en œuvre avec les moyens existants sur la ligne de production.

### Exemple 3 (selon l'invention)

Dans cet exemple, c'est de la paille qu'on traite avec le procédé de conversion de biomasse lignocellulosique en éthanol selon l'invention. La paille utilisée a les caractéristiques suivantes : Charge : Paille native, débit 3,36 tonne / heure, teneur en MS : 85,6%, composition moyenne (base MS) :

| | % (base MS) |
|---|---|
| Cellulose | 36,0% |
| Hémicellulose | 26,8% |
| Lignine et autres (cendres, extractibles, etc...) | 37,2% |

La paille est broyée sur une grille 50 mm, puis convoyée vers le réacteur d'imprégnation 5 par la vis conique 6a. Dans le réacteur d'imprégnation 5 sont introduites :
- la paille compressée, via la vis de transfert conique 6a
- 14,53 tonne / heure d'une liqueur acide préparée dans la cuve 3 de préparation de liqueur acide, à une température de 75°C
- 0,57 tonne / heure de cette même liqueur acide introduite en fond de réacteur d'imprégnation à une température de 75°C, dans la zone 5d sous le point d'introduction de la biomasse dans le réacteur.

La paille imprégnée est sortie du réacteur d'imprégnation 5 et transférée vers une vis de transfert 8 conique assurant son introduction dans un réacteur d'explosion à la vapeur 9. Lors du passage de la paille imprégnée dans la vis de transfert conique, un jus appelé pressât est extrait, à un débit moyen de 9,74 tonne/h, ce pressât se mélange avec l'eau de lavage externe de la vis conique, le débit total collecté est de 10,59 tonne/h, dont 85% sont recyclé. Le prétraitement par explosion à la vapeur est effectué à 185°C dans une configuration continue employant un court temps de séjour. Le milieu est brusquement détendu à une pression de 1,3 ATM.

La liqueur acide envoyée vers le réacteur d'imprégnation est préparée dans une cuve de liqueur acide en mélangeant :
- le pressât partiellement recyclé, à un débit de 9 tonne/h
- un apport d'acide H2SO4 à 96%pds, à un débit de 0,126 tonne heure
- un appoint en eau à un débit de 5,973 tonne / h

Le pressât 10 est extrait de la vis d'alimentation du réacteur d'explosion de prétraitement 9, et, dans cet exemple, il est collecté et pompé vers la cuve de préparation de liqueur 3 pour y être recyclé. La cuve de mélange 3 est donc ici alimentée par une conduite d'appoint en eau 1, une conduite 2 d'appoint en solution concentrée d'acide H₂SO₄, et une conduite supplémentaire amenant le pressât recyclé 10 (non représentée aux figures).

Le substrat prétraité ainsi obtenu à une teneur en xylose libre de 15,9% de xylose (base MS), et une teneur en xylose potentiel (monomère, oligomères et polymères) de 21,8% exprimé sous forme xylose (base MS).

Le substrat prétraité est ensuite envoyé vers le réacteur 16 d'hydrolyse enzymatique et de fermentation simultanées. Pour atteindre les conditions opératoires souhaitées, un flux de cocktail enzymatique, un flux de levures et un flux d'eau sont ajouté au substrat, pour un total de 4,85 tonne / h. Le cocktail enzymatique a été produit par un champignon *Trichoderma reesei,* la levure utilisée est une levure de type *Saccharomyces cerevisiae* génétiquement modifiée pour consommer le xylose. En fin de réaction, le milieu contient de l'éthanol à une concentration de 47 g / kg. Le vin 19 est envoyé dans une unité de séparation 20 permettant d'obtenir un flux d'éthanol concentré à 99,7%pds par distillation et déshydratation. Ce flux d'éthanol à un débit de 0,552 tonne / h.

L'injection de liqueur supplémentaire en fond de réacteur d'imprégnation est également efficace pour des biomasses du type paille.

## Revendications

1. Procédé de traitement d'une biomasse lignocellulosique (6), ledit procédé comprenant les étapes suivantes :
a. Préparation d'une liqueur d'imprégnation (4) contenant un catalyseur chimique destinée à l'imprégnation de la biomasse, choisi parmi un catalyseur acide, un catalyseur basique et un catalyseur oxydant, de préférence un catalyseur acide
b. Introduction de la biomasse broyée par une entrée d'un réacteur d'imprégnation (5), ladite entrée étant située dans une première zone d'imprégnation (5a) dudit réacteur d'imprégnation qui comprend deux zones superposées , ladite première zone d'imprégnation et une deuxième zone dite d'égouttage (5b) au-dessus de la zone d'imprégnation
c. Introduction de la liqueur (4) par une première entrée de liqueur située dans ladite première zone d'imprégnation (5a) du réacteur,
**caractérisé en ce qu'**il comprend également l'étape suivante :
d. Introduction de ladite liqueur dans ledit réacteur (5) par une deuxième entrée de liqueur dans une autre zone (5d) du réacteur située en dessous de l'entrée de la biomasse (6) située dans la première zone d'imprégnation (5b).

2. Procédé selon la revendication précédente, **caractérisé en ce que** la biomasse (6) est transportée de la première zone d'imprégnation (5a) à la deuxième zone d'égouttage (5b) par une ou des vis de transport (5c), la biomasse s'imprégnant de liqueur dans la première zone et s'égouttant dans la deuxième zone.

3. Procédé selon l'une des revendications précédentes, **caractérisé en ce que** l'introduction de la biomasse (6) dans la première zone d'imprégnation(5a) du réacteur est effectuée par un moyen d'alimentation (6a) créant un bouchon de biomasse (6b) empêchant la remontée de liquide depuis ladite première zone (5a) dans ledit moyen d'alimentation (6a), ledit moyen d'alimentation étant notamment une vis d'alimentation.

4. Procédé selon l'une des revendications précédentes, **caractérisé en ce que** 80 à 98%, notamment 85 à 90%, de la liqueur introduite dans le réacteur d'imprégnation (5) est introduite par la première entrée de liqueur (4a), et **en ce que** le complément à 100% de la liqueur introduite dans le réacteur est introduite par la deuxième entrée de liqueur (4b).

5. Procédé selon l'une des revendications précédentes, **caractérisé en ce que** l'introduction de liqueur par la première entrée de liqueur (4a) et/ou par la deuxième entrée de liqueur (4b) se fait de façon continue ou discontinue.

6. Procédé selon l'une des revendications précédentes, **caractérisé en ce que** l'autre zone (5d) du réacteur (5) dans laquelle est située la deuxième entrée de liqueur est une zone inactive située dans le fond du réacteur disposé sensiblement à la verticale ou à l'oblique par rapport à la verticale.

7. Procédé selon l'une des revendications précédentes, **caractérisé en ce que** la liqueur (4) est une liqueur à catalyse acide, et **en ce qu'**on règle le pH de la liqueur entre 0,1 et 4, notamment entre 0,3 et 2.

8. Procédé de traitement en continu d'une biomasse lignocellulosique, **caractérisé en ce qu'**il comprend les étapes selon l'une des revendications précédentes, et **en ce qu'**il se poursuit, en continu, en comprenant les étapes suivantes :
e. Transfert de la biomasse imprégnée et égouttée depuis la sortie du réacteur d'imprégnation (5) vers un réacteur de pré traitement par cuisson (9)
f. Pré-traitement de ladite biomasse dans ledit réacteur par cuisson (9)
g. Hydrolyse enzymatique (16) de la biomasse prétraitée (15)
h. Fermentation alcoolique (18) du moût d'hydrolyse enzymatique (17) obtenu à partir de la biomasse prétraitée.

9. Installation de traitement d'une biomasse lignocellulosique, comprenant :
- Une zone de préparation (3) d'une liqueur d'imprégnation (4) contenant un catalyseur chimique pour l'imprégnation de la biomasse, choisi parmi un catalyseur acide, basique ou oxydant, et de préférence un catalyseur acide, et munie d'une sortie de liqueur
- Un réacteur d'imprégnation (5) comprenant une première zone d'imprégnation (5a) munie d'une entrée de biomasse et une deuxième zone superposée à la première, dite zone d'égouttage (5b), et munie d'une sortie de biomasse
- Un moyen d'alimentation en biomasse broyée (6a) du réacteur d'imprégnation (5) par l'entrée de biomasse du réacteur située dans la première zone d'imprégnation (5a)
- Un premier moyen d'alimentation en liqueur d'imprégnation (4a) du réacteur (5) reliant une sortie en liqueur de la zone de préparation (3) de la liqueur (4) à une première entrée de liqueur dans la première zone d'imprégnation du réacteur,
**caractérisé en ce que** l'installation comprend également :
- Un deuxième moyen d'alimentation en liqueur d'imprégnation (4b) du réacteur (5) reliant une sortie en liqueur de la zone de préparation (3) de la liqueur à une deuxième entrée de liqueur dans une zone du réacteur (5d) située en dessous de l'entrée de la biomasse de la première zone d'imprégnation (5a).

10. Installation selon la revendication précédente, **caractérisée en ce que** le réacteur d'imprégnation (5) est d'orientation verticale ou oblique par rapport à la verticale, la biomasse effectuant un mouvement ascendant depuis la première zone d'imprégnation (5a) vers la deuxième zone d'égouttage (5b) par une ou des vis de transport (5c) disposées dans le réacteur dans lesdites zones.

11. Installation selon l'une des revendications 9 ou 10, **caractérisée en ce que** la zone de préparation de la liqueur d'imprégnation est une cuve (3) ou un mélangeur statique ou un mélangeur dynamique.

12. Installation selon l'une des revendications 9 à 11, **caractérisée en ce que** le premier et le deuxième moyens d'alimentation en liqueur comprennent une portion de conduite commune (4) reliée à une même sortie de liqueur de la zone de préparation (3) de la liqueur ou sont reliés chacun à une sortie distincte de la zone de préparation (3) de la liqueur.

13. Installation selon l'une des revendications 9 à 12, **caractérisé en ce que** le moyen d'alimentation en biomasse (6a) crée un bouchon de biomasse (6b) empêchant la remontée de liquide depuis ladite première zone dans ledit moyen d'alimentation, ledit moyen d'alimentation étant notamment une vis d'alimentation.

14. Installation selon l'une des revendications 9 à 13, **caractérisée en ce qu'**elle comprend également un réacteur de prétraitement (9) de la biomasse obtenue en sortie du réacteur d'imprégnation (5), un réacteur d'hydrolyse enzymatique (16) et un réacteur de fermentation alcoolique (18), l'ensemble des réacteurs étant montés en série, ou au moins deux d'entre eux.

15. Utilisation du procédé ou de l'installation selon l'une des revendications précédentes pour le traitement de biomasses (6) du type bois, paille, résidus agricoles, et toutes cultures énergétiques dédiées, notamment des plantes annuelles ou pluriannuelles telles que le miscanthus en vue de produire des sucres, des biocarburants ou des molécules bio-sourcées.

## Patentansprüche

1. Verfahren zur Behandlung von Biomasse aus Lignocellulose (6), wobei das Verfahren die folgenden Schritte umfasst:
a. Zubereitung einer Imprägnierflüssigkeit (4), die einen chemischen Katalysator enthält, der zur Imprägnierung der Biomasse bestimmt ist und aus einem sauren Katalysator, einem basischen Katalysator und einem oxidierenden Katalysator ausgewählt ist, vorzugsweise einen sauren Katalysator,
b. Einführung der zerkleinerten Biomasse durch einen Einlass eines Imprägnierreaktors (5), wobei sich der Einlass in einer ersten Imprägnierzone (5a) des Imprägnierreaktors befindet, der zwei übereinanderliegende Zonen umfasst, die erste Imprägnierzone und eine zweite sogenannte Abtropfzone (5b) oberhalb der der Imprägnierzone,
c. Einführung der Flüssigkeit (4) durch einen ersten Flüssigkeitseinlass, der sich in der ersten Imprägnierzone (5a) des Reaktors befindet,
**dadurch gekennzeichnet, dass** es auch den folgenden Schritt umfasst:
d. Einführung der Flüssigkeit in den Reaktor (5) durch einen zweiten Flüssigkeitseinlass in einer anderen Zone (5d) des Reaktors, die sich unterhalb des Einlasses der Biomasse (6) befindet, der sich in der ersten Imprägnierzone (5b) befindet.

2. Verfahren nach dem vorhergehenden Anspruch, **dadurch gekennzeichnet, dass** die Biomasse (6) durch eine oder mehrere Förderschnecken (5c) von der ersten Imprägnierzone (5a) in die zweite Abtropfzone (5b) befördert wird, wobei die Biomasse sich in der ersten Zone mit Flüssigkeit imprägniert und in der zweiten Zone abtropft.

3. Verfahren nach einem der vorhergehenden Ansprüche, **dadurch gekennzeichnet, dass** die Einführung der Biomasse (6) in die erste Imprägnierzone (5a) des Reaktors durch ein Zuführmittel (6a) erfolgt, das einen Biomassestopfen (6b) erzeugt, der das Wiederaufsteigen von Flüssigkeit aus der ersten Zone (5a) in das Zuführmittel (6a) verhindert, wobei das Zuführmittel insbesondere eine Zuführschnecke ist.

4. Verfahren nach einem der vorhergehenden Ansprüche, **dadurch gekennzeichnet, dass** 80 bis 98 %, insbesondere 85 bis 90 %, der Flüssigkeit, die in den Imprägnierreaktor (5) eingeführt wird, durch den ersten Flüssigkeitseinlass (4a) eingeführt wird und dass das Komplement zu 100 % der Flüssigkeit, die in den Reaktor eingeführt wird, durch den zweiten Flüssigkeitseinlass (4b) eingeführt wird.

5. Verfahren nach einem der vorhergehenden Ansprüche, **dadurch gekennzeichnet, dass** die Einführung von Flüssigkeit durch den ersten Flüssigkeitseinlass (4a) und/oder durch den zweiten Flüssigkeitseinlass (4b) kontinuierlich oder diskontinuierlich erfolgt.

6. Verfahren nach einem der vorhergehenden Ansprüche, **dadurch gekennzeichnet, dass** die andere Zone (5d) des Reaktors (5), in der sich der zweite Flüssigkeitseinlass befindet, eine inaktive Zone ist, die sich im Boden des Reaktors befindet, der im Wesentlichen vertikal oder schräg zur Vertikalen angeordnet ist.

7. Verfahren nach einem der vorhergehenden Ansprüche, **dadurch gekennzeichnet, dass** die Flüssigkeit (4) eine saure Katalyseflüssigkeit ist und dass der pH-Wert der Flüssigkeit zwischen 0,1 und 4 eingestellt wird, insbesondere zwischen 0.3 und 2.

8. Verfahren zur kontinuierlichen Behandlung von Biomasse aus Lignocellulose, **dadurch gekennzeichnet, dass** es die Schritte nach einem der vorhergehenden Ansprüche umfasst und dass es sich kontinuierlich fortsetzt, indem es die folgenden Schritte umfasst:
e. Transfer der imprägnierten und abgetropften Biomasse vom Auslass des Imprägnierreaktors (5) zu einem Hitzevorbehandlungsreaktor (9),
f. Vorbehandlung der Biomasse im Hitzereaktor (9),
g. Enzymatische Hydrolyse (16) der vorbehandelten Biomasse (15),
h. Alkoholische Gärung (18) der Maische aus der enzymatischen Hydrolyse (17), die auf der Grundlage der vorbehandelten Biomasse erhalten wurde.

9. Anlage zur Behandlung von Biomasse aus Lignocellulose, umfassend:
- eine Zubereitungszone (3) für eine Imprägnierflüssigkeit (4), die einen chemischen Katalysator zur Imprägnierung der Biomasse enthält, der aus einem sauren Katalysator, einem basischen Katalysator und einem oxidierenden Katalysator ausgewählt ist, vorzugsweise einen sauren Katalysator, und die mit einem Flüssigkeitsauslass versehen ist,
- einen Imprägnierreaktor (5), umfassend eine erste Imprägnierzone (5a), die mit einem Biomasseeinlass versehen ist, und eine zweite, über der ersten liegende Zone, die sogenannte Abtropfzone (5b), die mit einem Biomasseauslass versehen ist,
- ein Mittel zur Zuführung zerkleinerter Biomasse (6a) zum Imprägnierreaktor (5) durch den Biomasseeinlass des Reaktors, der sich in der ersten Imprägnierzone (5a) befindet,
- ein erstes Mittel zur Zuführung von Imprägnierflüssigkeit (4a) zum Reaktor (5), das einen Flüssigkeitsauslass der Zubereitungszone (3) für die Flüssigkeit (4) mit einem ersten Flüssigkeitseinlass in der ersten Imprägnierzone des Reaktors verbindet,
**dadurch gekennzeichnet, dass** die Anlage auch umfasst:
- ein zweites Mittel zur Zuführung von Imprägnierflüssigkeit (4b) zum Reaktor (5), das einen Flüssigkeitsauslass der Zubereitungszone (3) für die Flüssigkeit mit einem zweiten Flüssigkeitseinlass in einer Reaktorzone (5d) verbindet, die sich unterhalb des Biomasseeinlasses der ersten Imprägnierzone (5a) befindet.

10. Anlage nach dem vorhergehenden Anspruch, **dadurch gekennzeichnet, dass** der Imprägnierreaktor (5) eine vertikal oder schräg zur Vertikalen verlaufende Ausrichtung aufweist, wobei die Biomasse über eine oder mehrere Förderschnecken (5c), die im Reaktor in den Zonen angeordnet sind, eine aufsteigende Bewegung von der ersten Imprägnierzone (5a) zur zweiten Abtropfzone (5b) durchführt.

11. Anlage nach einem der Ansprüche 9 oder 10, **dadurch gekennzeichnet, dass** die Zubereitungszone der Imprägnierflüssigkeit ein Bottich (3) oder ein statischer Mischer oder ein dynamischer Mischer ist.

12. Anlage nach einem der Ansprüche 9 bis 11, **dadurch gekennzeichnet, dass** das erste und das zweite Mittel zur Zuführung von Flüssigkeit einen gemeinsamen Leitungsabschnitt (4) umfassen, der mit demselben Flüssigkeitsauslass der Zubereitungszone (3) für die Flüssigkeit verbunden ist, oder jeweils mit einem anderen Auslass der Zubereitungszone (3) für die Flüssigkeit verbunden sind.

13. Anlage nach einem der Ansprüche 9 bis 12, **dadurch gekennzeichnet, dass** das Mittel zur Zuführung von Biomasse (6a) einen Biomassestopfen (6b) erzeugt, der das Wiederaufsteigen von Flüssigkeit aus der ersten Zone in das Zuführmittel verhindert, wobei das Zuführmittel insbesondere eine Zuführschnecke ist.

14. Anlage nach einem der Ansprüche 9 bis 13, **dadurch gekennzeichnet, dass** sie auch einen Vorbehandlungsreaktor (9) für die Biomasse, die am Auslass des Imprägnierreaktors (5) erhalten wird, einen Reaktor für enzymatische Hydrolyse (16) und einen Reaktor für alkoholische Gärung (18) umfasst, wobei die Gesamtheit der Reaktoren oder mindestens zwei davon in Reihe angeordnet sind.

15. Nutzung des Verfahrens oder der Anlage nach einem der vorhergehenden Ansprüche zur Behandlung von Biomassen (6) vom Typ Holz, Stroh, landwirtschaftliche Reststoffe und jeglicher zweckbestimmter Energiekulturen, insbesondere einjährige oder mehrjährige Pflanzen wie etwa Miscanthus zum Zweck der Herstellung von Zuckern, Biokraftstoffen oder Molekülen auf Biobasis.

## Claims

1. A process for treating a lignocellulosic biomass (6), said process comprising the following steps:
a. Preparing an impregnation liquor (4) containing a chemical catalyst intended for the impregnation of the biomass, chosen from an acid catalyst, a basic catalyst and an oxidizing catalyst, and preferably an acid catalyst
b. Introducing the milled biomass via an inlet of an impregnation reactor (5), said inlet being located in a first impregnation zone (5a) of said impregnation reactor which comprises two superposed zones, said first impregnation zone and a second "draining" zone (5b) above the impregnation zone
c. Introducing the liquor (4) via a first liquor inlet located in said first impregnation zone (5a) of the reactor,
**characterized in that** it also comprises the following step:
d. Introducing said liquor into said reactor (5) via a second liquor inlet in another reactor zone (5d) located below the biomass inlet (6) located in the first impregnation zone (5b).

2. The process as claimed in the preceding claim, **characterized in that** the biomass (6) is transported from the first impregnation zone (5a) to the second draining zone (5b) by one or more transportation screws (5c), the biomass being impregnated with liquor in the first zone and being drained in the second zone.

3. The process as claimed in one of the preceding claims, **characterized in that** the introduction of the biomass (6) into the first impregnation zone (5a) of the reactor is performed by a feed means (6a) creating a biomass plug (6b) which prevents the backflow of liquid from said first zone (5a) into said feed means (6a), said feed means notably being a feed screw.

4. The process as claimed in one of the preceding claims, **characterized in that** 80% to 98%, notably 85% to 90%, of the liquor introduced into the impregnation reactor (5) is introduced via the first liquor inlet (4a), and **in that** the remainder to 100% of the liquor introduced into the reactor is introduced via the second liquor inlet (4b).

5. The process as claimed in one of the preceding claims, **characterized in that** the introduction of liquor via the first liquor inlet (4a) and/or via the second liquor inlet (4b) takes place continuously or discontinuously.

6. The process as claimed in one of the preceding claims, **characterized in that** the other zone (5d) of the reactor (5) in which the second liquor inlet is located is an inactive zone located at the bottom of the reactor positioned substantially vertically or obliquely relative to the vertical.

7. The process as claimed in one of the preceding claims, **characterized in that** the liquor (4) is an acid catalysis liquor, and **in that** the pH of the liquor is adjusted to between 0.1 and 4, notably between 0.3 and 2.

8. A process for the continuous treatment of a lignocellulosic biomass, **characterized in that** it comprises the steps as claimed in one of the preceding claims, and **in that** it is continued, continuously, by comprising the following steps:
e. Transferring the impregnated and drained biomass from the impregnation reactor outlet (5) to a cooking pretreatment reactor (9)
f. Pretreating said biomass in said cooking reactor (9)
g. Enzymatic hydrolysis (16) of the pretreated biomass (15)
h. Alcoholic fermentation (18) of the enzymatic hydrolysis must (17) obtained from the pretreated biomass.

9. A facility for treating a lignocellulosic biomass, comprising:
- A zone (3) for preparing an impregnation liquor (4) containing a chemical catalyst for the impregnation of the biomass, chosen from an acid, basic or oxidizing catalyst, and preferably an acid catalyst, and equipped with a liquor outlet
- An impregnation reactor (5) comprising a first impregnation zone (5a) equipped with a biomass inlet and a second zone superposed on the first, known as the draining zone (5b), and equipped with a biomass outlet
- A means for feeding milled biomass (6a) to the impregnation reactor (5) via the biomass inlet of the reactor located in the first impregnation zone (5a)
- A first means for feeding impregnation liquor (4a) to the reactor (5) connecting a liquor outlet of the liquor (4) preparation zone (3) to a first liquor inlet in the first impregnation zone of the reactor,
**characterized in that** the facility also comprises:
- A second means for feeding impregnation liquor (4b) to the reactor (5) connecting a liquor outlet of the liquor preparation zone (3) to a second liquor inlet in a reactor zone (5d) located below the biomass inlet of the first impregnation zone (5a).

10. The facility as claimed in the preceding claim, **characterized in that** the impregnation reactor (5) is of vertical or oblique orientation relative to the vertical, the biomass undergoing an ascending motion from the first impregnation zone (5a) to the second draining zone (5b) by means of one or more transportation screws (5c) placed in the reactor in said zones.

11. The facility as claimed in one of claims 9 and 10, **characterized in that** the impregnation liquor preparation zone is a tank (3) or a static mixer or a dynamic mixer.

12. The facility as claimed in one of claims 9 to 11, **characterized in that** the first and the second liquor feed means comprise a common pipe portion (4) connected to the same liquor outlet of the liquor preparation zone (3) or are each connected to a different outlet of the liquor preparation zone (3).

13. The facility as claimed in one of claims 9 to 12, **characterized in that** the biomass feed means (6a) creates a biomass plug (6b) which prevents the backflow of liquid from said first zone into said feed means, said feed means notably being a feed screw.

14. The facility as claimed in one of claims 9 to 13, **characterized in that** it also comprises a pretreatment reactor (9) for the biomass obtained at the outlet of the impregnation reactor (5), an enzymatic hydrolysis reactor (16) and an alcoholic fermentation reactor (18), all of the reactors, or at least two of them, being mounted in series.

15. The use of the process or of the facility as claimed in one of the preceding claims, for the treatment of biomasses (6) such as wood, straw, agricultural residues, and all dedicated energy crops, notably annual or perennial plants such as miscanthus, in order to produce sugars, biofuels or biobased molecules.
